# EUROPEAN PATENT APPLICATION

(11) **EP 3 460 826 A1**
(43) Date of publication of application: **27.03.2019**
(21) Application number: 18194944.7
(22) Date of filing: 17.09.2018
(51) Int. Cl.: H01J 49/00, G01N 33/68

(54) **ANALYSIS METHOD AND ANALYTICAL DEVICE**

(30) Priority: 20.09.2017 JP 2017180297
(71) Applicant: Shimadzu Corporation, Kyoto 604-8511 (JP)
(72) Inventor: TAKAHASHI, Hidenori, Kyoto-shi,, Kyoto 6048511 (JP)
(74) Representative: Mewburn Ellis LLP

(57) **Abstract**

An analysis method includes: converting a first ion derived from a sample component into a radical to generate a second ion; reacting the second ion or an ion generated by dissociation of the second ion with an introduction gas to generate a third ion; detecting the third ion or an ion generated by dissociation of the third ion by mass analysis to obtain a first mass spectrum: and identifying a peak on the first mass spectrum based on a difference in mass between the second ion or the ion generated by dissociation of the second ion and the third ion or the ion generated by dissociation of the third ion due to the composition of the introduction gas.

## Description

The disclosure of the following priority application is herein incorporated by reference: Japanese Patent Application No. 2017-180297 filed September 20, 2017.

The present invention relates to an analysis method and an analytical device.

Mass spectrometry has been performed by ionizing a sample component to yield precursor ions, dissociating the precursor ions to generate various fragment ions (product ions), obtaining a mass spectrum showing peaks corresponding to the various fragment ions and analyzing the sample component using the mass spectrum. This analysis provides detailed information about the structure of a molecule contained in the sample component, for example, information about an amino acid sequence, from a plurality of fragment ions that constitute respective parts of the molecule of the sample component.

For example, analysis of a peptide involves generation of fragment ions that usually include various kinds of fragment ions, such as those derived from the N-terminal side, those derived from the C-terminal side, those derived from cleavage of N-Cα bond on the main chain of the peptide, and those derived from cleavage of side chains. Under the circumstances, mostly it is difficult to reach an assumed structure of a molecule based on the peaks on the mass spectrum. Accordingly, a method that is easier to understand and is more efficient has been sought.

One of the conventional methods proposed for solving this object involves identification of peaks on the mass spectrum by using characteristics of the fragment ions generated by a specified dissociation method. WO 2015/133259 A and an article by Takahashi, et al. (Hidenori Takahashi, Sadanori Sekiya, Takashi Nishikaze, Kei Kodera, Shinichi Iwamoto, Motoi Wada, and Koichi Tanaka, "Hydrogen Attachment/Abstraction Dissociation (HAD) of Gas-Phase Peptide Ions for Tandem Mass Spectrometry", Analytical Chemistry, (US), ACS Publications, March 22, 2016, Volume 88, Issue 7, pp.3810-3816) disclose generation of c-type fragment ions (c-ions) derived from the cleavage of N-Cα bonds, which are difficult to be generated by low energy Collision-Induced Dissociation (CID), by utilizing the specific reactivity of a hydrogen radical on the main chain of a peptide.

A further proposal for providing a method for generating fragment ions that allow for an easier analysis by utilizing characteristics, for example, dissociation, is desired to make a more efficient analysis of various molecules.

According to the 1st aspect of the present invention, an analysis method comprises: converting a first ion derived from a sample component into a radical to generate a second ion; reacting the second ion or an ion generated by dissociation of the second ion with an introduction gas to generate a third ion; detecting the third ion or an ion generated by dissociation of the third ion by mass analysis to obtain a first mass spectrum: and identifying a peak on the first mass spectrum based on a difference in mass between the second ion or the ion generated by dissociation of the second ion and the third ion or the ion generated by dissociation of the third ion due to the composition of the introduction gas.

According to the 2nd aspect of the present invention, in the analysis method according to the 1st aspect, it is preferred that the method further comprises: detecting the ion generated by dissociation of the second ion by mass analysis and forming a second mass spectrum; and identifying corresponding peaks in the first mass spectrum and the second mass spectrum based on a difference in mass between the second ion or the ion generated by dissociation of the second ion and the third ion or the ion generated by dissociation of the third ion due to the composition of the introduction gas.

According to the 3rd aspect of the present invention, in the analysis method according to the 1st or 2nd aspect, it is preferred that the third ion or the ion generated by dissociation of the third ion is generated by attachment of a portion of a molecule contained in the introduction gas to the second ion or the ion generated by the second ion.

According to the 4th aspect of the present invention, in the analysis method according to any one of the 1st through 3rd aspects, it is preferred that the sample component is at least one molecule selected from the group consisting of amino acid, peptide, and protein; and the introduction gas contains a molecule that comprises an oxygen atom.

According to the 5th aspect of the present invention, in the analysis method according to 4th aspect, it is preferred that an a-type ion is identified based on a difference in mass between the second ion or the ion generated by dissociation of the second ion and the third ion or the ion generated by dissociation of the third ion due to attachment of the oxygen atom.

According to the 6th aspect of the present invention, in the analysis method according to any one of the 1st through 3rd aspects, it is preferred that the sample component comprises a side chain that contains a fatty acid or hydrocarbon moiety; and the introduction gas contains a molecule that comprises an oxygen atom.

According to the 7th aspect of the present invention, in the analysis method according to 6th aspect, it is preferred that the sample component is identified to be derived from a lipid based on a difference in mass between the second ion or the ion generated by dissociation of the second ion and the third ion or the ion generated by dissociation of the third ion due to attachment of the oxygen atom.

According to the 8th aspect of the present invention, in the analysis method according to any one of the 1st through 7th aspects, it is preferred that the first ion is converted to a radical by reacting the first ion with a gas containing a radical.

According to the 9th aspect of the present invention, in the analysis method according to 8th aspect, it is preferred that the radical is a hydrogen radical, and the second ion is generated by attachment of the hydrogen radical to the first ion or by abstraction of the hydrogen radical from the first ion.

According to the 10th aspect of the present invention, in the analysis method according to any one of the 1st through 7th aspects, it is preferred that the first ion is converted into a radical by irradiating the first ion with electrons.

According to the 11th aspect of the present invention, in the analysis method according to any one of the 1st through 10th aspects, it is preferred that the introduction gas contains an oxygen molecule.

According to the 12th aspect of the present invention, in the analysis method according to any one of the 1st through 11th aspects, it is preferred that the method further comprises: dissociating the third ion by any one of methods consisting of gas impact dissociation, laser irradiation dissociation, electronic ionization dissociation, electron transfer dissociation, and dissociation by attachment or abstraction of hydrogen to generate fragment ions.

According to the 13th aspect of the present invention, an analytical device comprises: an ion generating unit that converts a first ion derived from a sample component into a radical to generate a second ion, reacts the second ion or an ion generated by dissociation of the second ion with an introduction gas to generate a third ion or an ion generated by dissociation of the third ion; a detection unit that detects the third ion or the ion generated by the third ion by mass analysis; a first gas introduction unit that introduces a cooling gas and/or CID gas to the ion generating unit; and a second gas introduction unit that is configured to be controllable independently from control of the cooling gas and/or CID gas and introduces the introduction gas to the ion generating unit.

According to the 14th aspect of the present invention, in the analysis device according to 13th aspect, it is preferred that the device further comprises: an analysis unit that identifies a peak in a mass spectrum detected by the mass analysis based on a difference in mass between the second ion or the ion generated by dissociation of the second ion and the third ion or the ion generated by dissociation of the third ion due to the composition of the introduction gas.

According to the 15th aspect of the present invention, in the analytical device according to the 13th or 14th aspect, it is preferred that the device further comprises: a radical introduction unit that introduces a radical, wherein the ion generating unit converts the first ion into a radical by reaction of the first ion with a gas containing a radical.

According to the 16th aspect of the present invention, in the analysis device according to 15th aspect, it is preferred that the radical is a hydrogen radical, and the second ion is generated by attachment of the hydrogen radical to the first ion or by abstraction of the hydrogen radical from the first ion.

According to the 17th aspect of the present invention, in the analytical device according to the 13th or 14th aspect, it is preferred that the device further comprises: an electron irradiation unit that irradiates electrons, wherein the ion generating unit converts the first ion into a radical by irradiating the first ion with electrons.

According to the 18th aspect of the present invention, in the analytical device according to any one of the 13th through 17th aspects, it is preferred that the introduction gas contains a molecule that comprises an oxygen atom.

### BRIEF DESCRIPTION OF THE DRAWINGS

FIG. 1 is a diagram for illustrating a schematic configuration of the analytical device related to the analysis method according to an embodiment.
FIG. 2 is a diagram for illustrating an example of the reaction that occurs in the analysis method according to an embodiment.
FIG. 3 is a flowchart for illustrating the processing flow of the analysis method according to an embodiment.
FIG. 4 is a diagram for illustrating an example of the reaction that occurs in the analysis method according to a variation.
FIG. 5 is a diagram for illustrating a schematic configuration of the analytical device related to a variation.
FIG. 6 is a flowchart for illustrating the processing flow of the analysis method according to a variation.
FIG. 7 is a diagram for illustrating mass spectra concerning the mass spectroscopy of a peptide conducted in an example.
FIG. 8 is a diagram for illustrating the mass spectra of a lipid ion concerning the mass spectroscopy conducted in an example.
FIG. 9 is a diagram for illustrating the mass spectra concerning the mass spectroscopy of a peptide conducted in an example.

### DESCRIPTION OF EMBODIMENTS

Hereinafter, an embodiment of the present invention is explained with reference to the attached drawings.

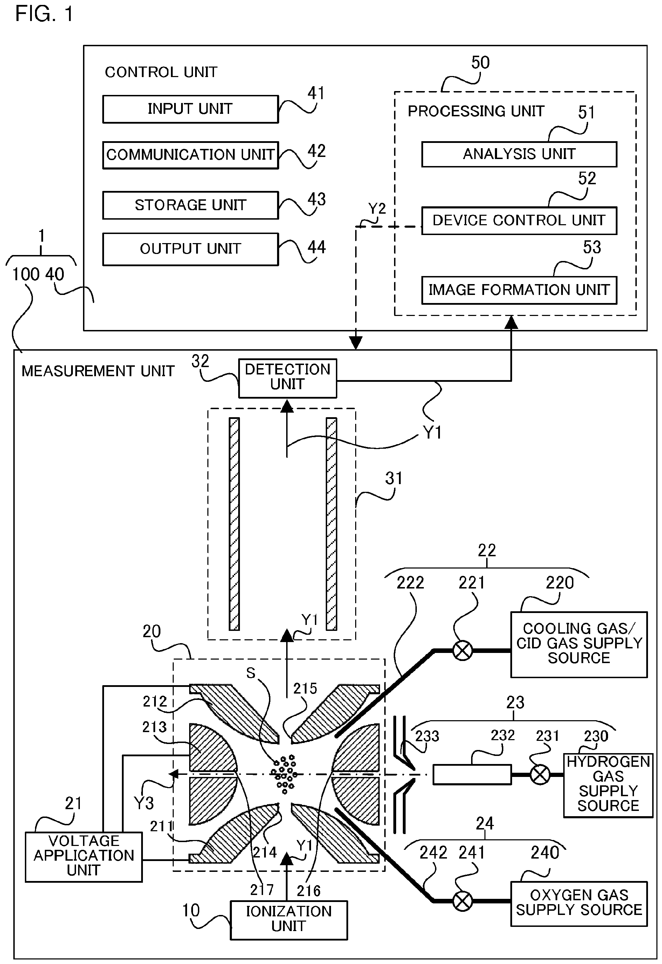

FIG. 1 is a diagram for illustrating a schematic configuration of an analytical device 1 related to an analysis method according to the embodiment. The analytical device 1 includes a measurement unit 100 and a control unit (controller) 40.

The measurement unit 100 includes an ionization unit 10, an ion generation unit (ion generator) 20 that stores ions S derived from a sample, a voltage application unit 21, a cooling gas/CID gas supply unit (cooling gas/CID gas supplier) 22, a radical supply unit (radical supplier) 23, an oxygen gas supply unit (oxygen gas supplier) 24, a time-of-flight mass separation unit (time-of-flight mass separator) 31, and a detection unit (detector) 32.

The ion generation unit 20 includes an inlet side end cap electrode 211 and an outlet side end cap electrode 212, a ring electrode 213, an ion introduction port 214, an ion ejection port 215, a radical introduction unit 216, and a radical discharge unit 217. The cooling gas/CID gas supply unit 22 includes a cooling gas/CID gas supply source 220, a valve 221, and a cooling gas/CID gas introduction unit 222. The radical supply unit 23 includes a hydrogen gas supply source 230, a valve 231, a radical generation unit (radical generator) 232, and a radical separation unit (radical separator) 233. The oxygen gas supply unit (oxygen gas supplier) 24 includes an oxygen gas supply source 240, a valve 241, and an oxygen gas introduction unit 242.

The control unit 40 includes an input unit 41, a communication unit 42, a storage unit 43, an output unit 44, and a processing unit 50. The processing unit 50 includes an analysis unit 51, a device control unit 52, and an image formation unit 53. FIG. 1 schematically illustrates a movement path of ions derived from a sample component to be detected and flow of detection signals of these ions with solid line arrows Y1 and control of the measurement unit 100 by the device control unit 52 with a broken line arrow Y2. Also, FIG. 1 illustrates the direction of movement of hydrogen radicals supplied from the radical supply unit 23 with a chain line arrow Y3. Apart or all of the functions of the control unit 40 may be implemented on an electric computer or the like which is physically separated from the measurement unit 100.

The ionization unit 10 of the measurement unit 100 includes an ion source and ionizes a sample component. The method of ionization is not particularly limited to any specific method and may be a MALDI method, for example. Ions S derived from the sample component that has been ionized at the ionization unit 10 are introduced into the ion generation unit 20 through an ion introduction port 214.

The ion generation unit 20 includes a vacuum chamber that can hold ions, such as an ion trap, in which the ions S derived from the sample component are reacted with a gas introduced into the ion generation unit 20 to change the structure of the ions derived from the sample component or generate fragment ions. The ion generation unit 20 controls the ions S derived from the sample component by means of voltages applied to the end cap electrodes 211 and 212 and the ring electrode 213 by the voltage application unit 21 and by means of an introduced cooling gas so that the ions S can be held or discharged as appropriate.

The ion generation unit 20 allows the ions S derived from the sample component and introduced thereinto to react with hydrogen radicals introduced from the radical supply unit 23 through a radical introduction unit 216 to generate radical ions obtained by radical formation of the ions S derived from the sample component.

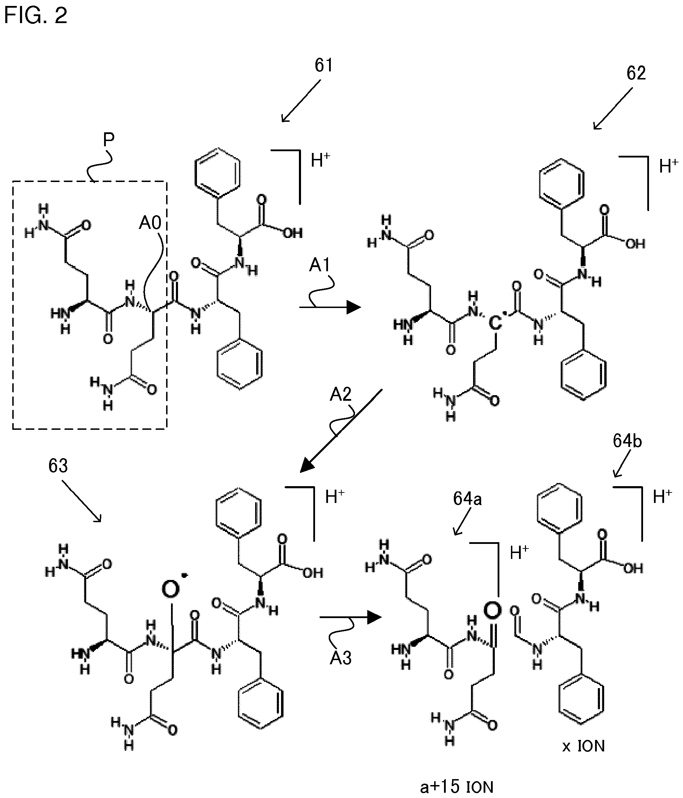

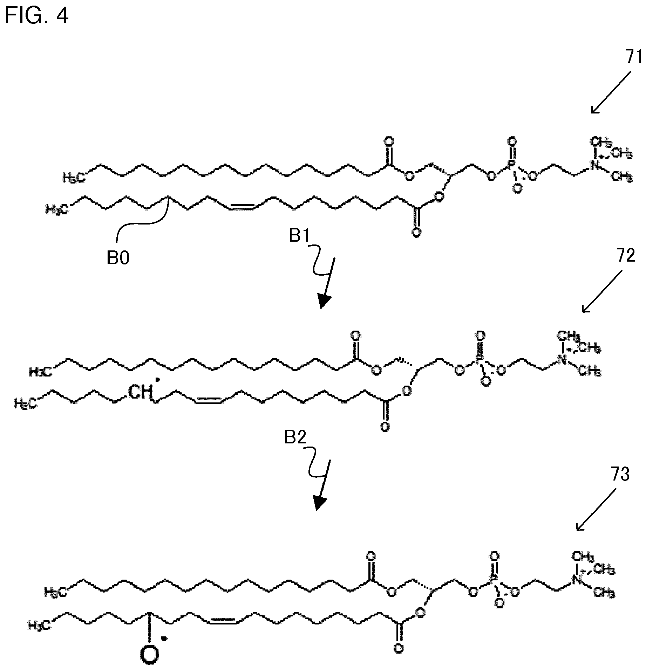

FIG. 2 is a diagram for illustrating a radical reaction that occurs in the ion generation unit 20. FIG. 2 and FIG. 4 described hereinbelow are explanation diagrams and the molecules presented therein are not intended to limit the present invention. A peptide 61 illustrated in FIG. 2 is presented as an example of the precursor ion S that is derived from the sample component and serves as a substrate in the radical reaction. Irradiating the peptide 61 with a stream of hydrogen radicals causes a hydrogen atom to be abstracted from a Cα carbon designated by A0 on the main chain of the peptide 61 to allow the Cα carbon A0 to have an unpaired electron. As a result, a radical formation reaction can occur (arrow A1). As a result of this reaction, the peptide 61, which is the ion S derived from the sample component, becomes a radical ion 62. The radical ion 62 can be formed by attachment of hydrogen instead of abstraction of hydrogen. If a radical other than the hydrogen radical can react with the ion S derived from the sample component to covert the ion S into its radical, such radical other than hydrogen may be used in the reaction for converting the ion S into its radical.

The ion generation unit 20 is configured to react the radical ion 62 with oxygen gas introduced from the oxygen gas supply unit 24 through the oxygen gas introduction unit 242 to generate an ion that has a changed mass, in particular a changed mass number, as compared with the radical ion 62 (hereinafter, referred to as "mass-changed ion").

FIG. 2 shows chemical formulas of ions involved in the generation of a mass-changed ion 63 from the radical ion 62. The Cα carbon that has been subjected to radical formation when the radical ion 62 was generated from the peptide 61 has increased reactivity due to the presence of an unpaired electron therein, which reactivity is high enough for an oxygen atom to be abstracted from an oxygen molecule contained in the oxygen gas (see arrow A2). The abstracted oxygen atom is attached to the Cα carbon that has the unpaired electron through a single covalent bond to produce a radical that has an unpaired electron.

The gas that can be used in the reaction in which the mass-changed ion 63 is generated (arrow A2) is not limited to oxygen gas and a gas having various compositions (hereafter, referred to as "introduction gas") may be used. The introduction gas may be constituted with a molecule having low reactivity such as a non-radical molecule having no unpaired electron since the high reactivity of the radical ion 62 is available. The composition of the introduction gas is not particularly limited as far as it contains a molecule that can react with the radical ion 62 obtained by radical formation of the ion S derived from the sample component and a difference in mass and/or m/z between the radical ion 62 and the mass-changed ion 63 caused by the reaction can be derived. The introduction gas may include one or more kinds of molecules that are selected from the group consisting of methane, ethane, ethylene, propane, propylene, hydrogen, nitrogen, oxygen, carbon monoxide, carbon dioxide, nitrous oxide, and ammonia. From the view point of easiness to handle and availability, oxygen gas is particularly preferred as the introduction gas.

At the mass-changed ion 63, the unpaired electron of the radical-converted oxygen atom and the electron of the Cα carbon that participates in binding on the main chain of the peptide move and together form a covalent bond between the oxygen atom and the Cα carbon. This produces a carbonyl bond between the oxygen atom and the Cα carbon and cuts the main chain of the peptide. As a result, the mass-changed ion 63 is cleaved into two fragment ions 64a and 64b (see arrow A3).

The fragment ion 64a labeled "a+15 ion" in FIG. 2 has an N-terminal of the peptide 61 and is an a-type ion (a-ion) generated by the cleavage of a Cα-C bond on the main chain of the peptide. Comparing the fragment ion 64a with the corresponding part P of the peptide 61, a hydrogen atom (mass number: 1) is absent by abstraction and an oxygen atom (mass number: 16) is present by attachment in the fragment ion 64a. As a result, the fragment ion 64a has a mass number by 15 larger than that of the corresponding part P of the peptide 61.

The fragment ion 64b labeled "x ion" in FIG. 2 has the C-terminal of the peptide 61 and is an x-type ion generated by the cleavage of the Cα-C bond on the main chain of the peptide 61. The fragment ion 64b is shown in FIG. 2 as having a hydrogen atom bonded to the cleaving site.

The reaction illustrated in FIG. 2 can produce an a-type ion, which has a known difference in mass from the mass of the a-series ion obtained by the cleavage of the peptide 61 (in the case of the reaction illustrated in FIG. 2, a change in mass based on the difference of a mass number of 15). A cleaved ion having a similar change in mass can be generated at each Cα carbon contained in the peptide 61. Therefore, the ion generation unit 20 can generate each a-type ion having caused therein a change in mass that can be derived taking each amino acid position k (k=1, 2, 3, ..., n-1, with n being the number of amino acids constituting the ion S derived from the sample) as a cleaving site (hereafter, referred to as "aₖ+15 ion").

The ion generation unit 20 generates, in addition to the aₖ+15 ion described above, an a-type ion using each amino acid position k as a cleaving site without using oxygen gas in the reaction (hereafter, referred to as "aₖ ion"). The method for generating aₖ ions is not particularly limited as far as the difference in mass between the aₖ ion and the aₖ+15 ion can be derived. As described later, each aₖ ion and each aₖ+15 ion (k=1, 2, ..., n-1) corresponding thereto will have peaks on a mass spectrum that are shifted one from another by an amount of the difference in m/z that corresponds to the difference in mass. Accordingly, by using this, the peaks of the a-type ions can be readily identified. As stated above, the analytical device 1 can suitably analyze a sample containing, as sample component, an amino acid, and a molecule that is formed by a plurality of amino acids bound together through peptide bonds, such as peptide and protein.

In the reaction illustrated in FIG. 2, an oxygen atom contained in the introduction gas is attached to the radical ion 62 to form the mass-changed ion 63 and subsequently the mass-changed ion 63 is cleaved. However, the cleavage may occur in any step of the reaction. That is, the ion generation unit 20 may be configured as follows. At the ion generation unit 20, the radical ion 62 is generated from the peptide 61, which is an ion S derived from a sample, and subsequently the radical-converted ion 62 is cleaved to generate a cleaved radical ion, such as a-type radical-converted ion. Subsequently, an atom contained in the introduction gas is attached to the cleaved radical ion to generate a mass-changed fragment ion, such as an aₖ+15 ion. In the following embodiment, the term "radical ion" indicates an ion derived from a sample before the mass is changed by a reaction with a molecule contained in the introduction gas regardless of whether cleavage occurs. The term "mass-changed ion" refers to an ion derived from a sample after the mass is changed by such a reaction.

Also, the ion generation unit 20 may be configured to perform dissociation of the mass-changed ion 63 by, for example, CID, instead of causing radical cleavage of the mass-changed ion 63 to occur due to a radical-converted atom therein as is observed in the reaction illustrated in FIG. 2. Like this, the mass-changed ion 63 can be dissociated by gas impact dissociation, laser irradiation dissociation, electron ionization dissociation, electron transfer dissociation, dissociation by attachment or abstraction of hydrogen or the like.

As illustrated in FIG. 1, the voltage application unit 21 includes a high-frequency power source and applies voltages to end-cap electrodes 211 and 212 and a ring electrode 213, thereby controlling movement of ions S that is derived from the sample and is introduced into the ion generating unit 20. An inlet side end-cap electrode 211 provided with an ion introducing port 214 is opposite to an outlet side end-cap electrode 212 provided with an ion ejection port 215 with an annular ring electrode 213 therebetween. By controlling voltages by the voltage application unit 21, the ions S derived from the sample are trapped in the ion generation unit 20 and fragment ions generated in the ion generation unit 20 are discharged to a time-of-flight mass separation unit 31 through the ion ejection port 215.

The cooling gas/CID gas supply unit 22 supplies a cooling gas and/or CID gas to the ion generation unit 20. The cooling gas/CID gas supply source 220 includes a cooling gas storage container (not shown in the figures) that contains a cooling gas, such as helium, and/or a CID gas storage container (not shown in the figures) that contains a CID gas, such as argon. The compositions of the cooling gas and the CID gas are not particularly limited. The introduction of the cooling gas and/or CID gas is controlled by opening and closing of the valve 221 controlled by the device control unit 52 described later, which is provided in the middle of the gas conduit for these gases. The cooling gas and/or CID gas introduction unit 222 is provided with a conduit that extends to the ion generation unit 20 and introduces a cooling gas and/or a CID gas into the ion generation unit 20. Although FIG. 1 schematically shows only one conduit as the cooling gas and/or CID gas introduction unit 222, the cooling gas and the CID gas may be introduced into the ion generation unit 20 through a plurality of different conduits separately.

The radical supply unit 23 supplies hydrogen radicals to the ion generation unit 20. The hydrogen gas supply source 230 is provided with a storage container (not shown in the figures) for a gas that contains hydrogen molecules to be converted into radicals. It is preferred that the molecules to be converted into radicals contain molecules that belong to one or more molecule groups selected from a group consisting of chlorides, sulfur compounds, fluorides, hydroxides, oxides, nitrides, and carbides. The molecules to be converted into radicals preferably include one or more kinds of molecules selected from the group consisting of hydrochloric acid, sodium chloride, sulfuric acid, sodium sulfate, fluoric acid, sodium fluoride, sodium hydrogen carbonate, sodium hydroxide, hydrogen peroxide, carbon dioxide, carbohydrates, and hydrocarbons. A particularly preferred molecule to be converted into a radical is hydrogen.

The discharge of the gas from the hydrogen supply source 230 is controlled by opening and closing of the valve 231 that is provided in the middle of the conduit for the hydrogen gas and controlled by the device control unit 52 described below. The gas that has passed the valve 231 is introduced into the radical generation unit 232. The radical generation unit 232 is provided with a radical generation device that includes a metal such as tungsten and an electric power source for applying current to the metal to heat it. For example, hydrogen radicals can be generated by passing a hydrogen gas through a tungsten capillary that is heated at a high temperature such as 2,000 °C. The hydrogen radicals generated at the radical generation device 232 are jetted through a nozzle or the like (not shown in the figures) into a radical separation unit 233 that has a skimmer or the like.

The radical separation unit 233 removes as appropriate at least a part of hydrogen radicals and hydrogen gas that are moving in a direction other than a direction toward the ion generation unit 20 and the separated hydrogen radicals are introduced into the radical introduction unit 216. The radical introduction unit 216 includes an orifice formed on the ring electrode 213 and introduce these introduced hydrogen radicals to ion generating unit 20. To enable efficient radical reaction to occur at the ion generation unit 20, it is preferred that the hydrogen radicals be introduced into a space in which the ion generation unit 20 is located at a flow rate of 4×10¹⁰ [atoms/s] or more. Alternatively, it is preferred to introduce hydrogen radicals into a space in which the ion generation unit 20 is located at a density of 3x10¹² [atoms/m³] or more.

The radical discharge unit 217 is formed on a line that is substantially the same as a long axis of the orifice at the radical introduction unit 216 and discharges hydrogen radicals and the like that have not undergone interaction with the ions S derived from the sample to prevent a decrease in the degree of vacuum. The methods of generating and introducing the radicals are not particularly limited to any specific ones so far as a sufficient flow rate of radicals being necessary to generate a sufficient amount of the radical ions 62 needed for detection of the fragment ions 64a illustrated in FIG. 2 is generated.

The oxygen gas supply unit 24 supplies an oxygen gas to the ion generation unit 20. The oxygen gas supply source 240 is provided with an oxygen gas storage container (not shown in the figures) that contains an oxygen gas. The introduction of the oxygen gas is controlled by opening and closing of the valve 241 that is provided in the middle of the conduit for the oxygen gas and controlled by the device control unit 52 described below. The oxygen gas introduction unit 242 is provided with a conduit that extends to the ion generation unit 20 and introduces the oxygen gas into the ion generating unit 20. The oxygen gas supply unit 24 may be configured to introduce the oxygen gas before irradiation of the hydrogen radicals to the ions S derived from the sample is performed, during such irradiation is continued or after such irradiation is completed.

The time-of-flight mass separation unit 31 is provided with a vacuum chamber in which ions fly, such as a flight tube, and separates the fragment ions that are ejected from the ion ejection port 215 and accelerated, according to the times of flight of the respective ions. The configuration of the time-of-flight mass separation unit 31 is not particularly limited to any specific one and a multi-turn type, a reflection type and so on in addition to the linear type as illustrated in the figure may be adopted as appropriate. The method of mass separation is not particularly limited to any specific one as far as the fragment ions generated at the ion generation unit 20 having different m/z values can be detected by separating them at a desired resolution. For example, the separation function by an ion trap of the ion generation unit 20 may also be used.

The detection unit 32 is provided with a charged particle detector, such as a microchannel plate, and detects the fragment ions separated according to their times of flight at the time-of-flight mass separation unit 31. The detection signals that are detected at the detection unit 32 are A/D converted by an A/D converter (not shown in the figures) and outputted to the processing unit 50.

The input unit 41 in the control unit 40 includes an input device such as a mouse, a keyboard, various types of buttons and/or touch panels. The input unit 41 receives, from the user, information that is necessary for controlling the operation of the measurement unit 100 and information that is necessary for processing that the processing unit 50 performs, and so on.

The communication unit 42 in the control unit 40 includes a communication device that enables communication via wireless connection such as the Internet or wired connection. It transmits results of analysis by the analysis unit 51 and an image formed by the image formation unit 53, and so on and transmits and receives necessary data as appropriate.

The storage unit 43 in the control unit 40 includes a nonvolatile storage medium and stores data relating to measurement results outputted from the measurement unit 100, a program for causing the processing unit 50 to execute processing, analysis results from the analysis unit 51, and images formed by the image formation unit 53, and so on.

The output unit 44 in the control unit 40 includes a display monitor such as a liquid crystal monitor or a printer and displays on the display monitor or prints on a paper medium the information relating to the measurement by the measurement unit 100, the analysis results by the analysis unit 51, the images formed by the image formation unit 53, and so on.

The processing unit 50 in the control unit 40 includes a microprocessor or a processor such as a CPU and functions as a subject that controls the analysis device 1. The processing unit 50 performs various types of processing by executing a program that is stored at the storage unit 43 or the like.

The analysis unit 51 in the processing unit 50 analyzes data outputted from the detection unit 32 (hereafter, referred to as "MS data") to quantify each fragment ion. From the MS data, the analysis unit 51 constructs a mass spectrum concerning fragment ions to be measured. The analysis unit 51 identifies a peak for each fragment ion on the mass spectrum based on information about a difference in mass between the radical ion 62 and the mass-changed ion 63 inputted from the input unit 41 (hereafter, referred to as "mass difference information"). Identification of peaks can be performed by calculating mass differences between peaks of supposed fragment ions by various methods based on the mass difference information and performing identification of the peaks based on the mass differences. For example, identification of peaks can be performed based on the difference in mass between the radical ion 62 and an ion obtained by dissociation of the mass-changed ion 63.

The analysis unit 51 on one hand determines the mass spectrum of the ion S derived from the sample without introducing oxygen gas into the ion generation unit 20 (hereafter, referred to "non-introduction mass spectrum") and on the other hand determines the mass spectrum of the ion S derived from the sample with introducing oxygen gas into the ion generation unit 20 (hereafter, referred to "introduction mass spectrum"). Then it compares these mass spectra with each other to identify corresponding peaks on the mass spectra. The analysis unit 51 analyzes these mass spectra based on the mass difference information.

The analysis of mass spectra is explained hereinbelow taking the reaction illustrated in FIG. 2 as an example. In the case of the peptide 61, the difference in mass between the mass-changed ion 63 and the radical ion 62 is 15 in mass number. Therefore, if the ion is monovalent, the peak of the aₖ+15 ion determined with introducing an oxygen gas corresponds to one that is obtained by shifting the peak of the aₖ ion determined without introducing an oxygen gas by 15 toward an increment side of m/z. If comparison of the mass spectra indicates that a peak on the non-introduction mass spectrum being shifted toward the increment side of m/z by 15 in mass number overlaps a peak on the introduction mass spectrum, the analysis unit 51 can specify the overlapping peaks to be peaks that corresponds to a-type ions. The method for identifying the peaks of mass spectra by using the mass difference information is not particularly limited to any specific one. The user may perform analysis reviewing the mass spectra displayed at the output unit 44.

The device control unit 52 of the processing unit 50 controls the operation of the measurement unit 100 based on the information relating to measurement conditions inputted by the input unit 41 or the information stored at the storage unit 43. The device control unit 52 controls the voltages to be applied by the voltage application unit 21, the supply of a cooling gas and/or CID gas, a radical gas, and an oxygen gas, detection conditions of the detection unit 32, and so on.

The image formation unit 53 of the processing unit 50 forms an output image from data corresponding to the mass spectrum based on MS data, the measuring conditions of the measurement unit 100 and/or analysis results of the analysis unit 51, and so on and outputs the formed output image to the output unit 44.

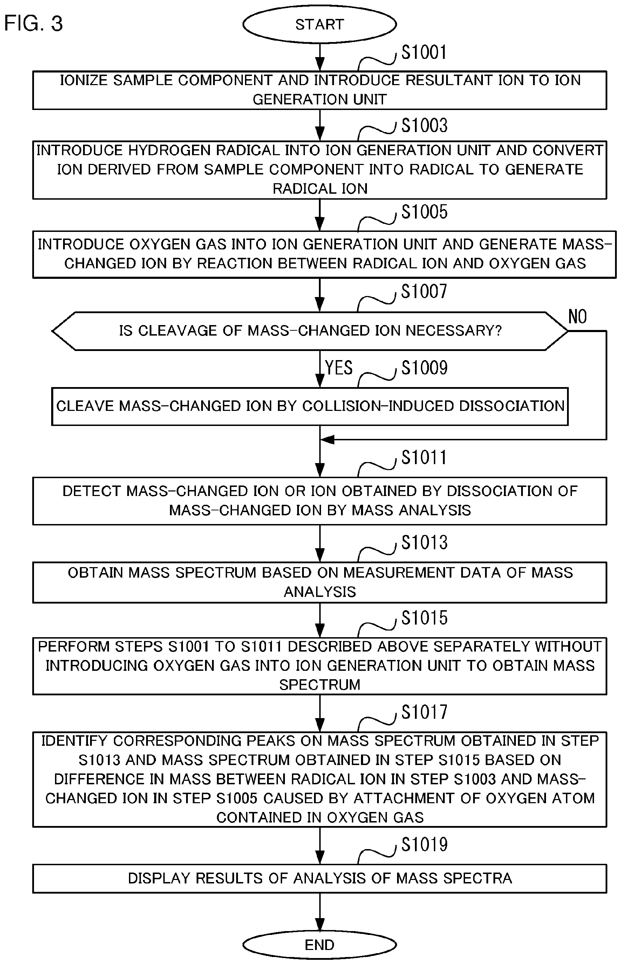

FIG. 3 is a flowchart illustrating the flow of processing of the analysis method according to the present embodiment. In step S1001, the ionization unit 10 ionizes a sample component and introduces the resultant ions into the ion generation unit 20. After step S1001 is completed, step S1003 is started.

In step S1003, the radical introduction unit 216 introduces hydrogen radicals into the ion generation unit 20 and the ion generating unit 20 converts the ions S derived from the sample component into radicals to generate the radical ions 62. After step S1003 is completed, step S1005 is started. In step S1005, the oxygen gas introduction unit 241 introduces an oxygen gas into the ion generation unit 20 and the ion generating unit 20 generates the mass-changed ions 63 by reaction between the radical ions 62 and the oxygen gas. After step S1005 is completed, step S1007 is started.

In step S1007, the processing unit 50 determines whether it is necessary to cleave the mass-changed ions 63 generated in step S1005 according to an instruction from the user that has been inputted from the input unit 41 in advance. When it is necessary to cleave the mass-changed ions 63, the processing unit 50 makes an affirmative determination on step S1007 and step S1009 is started. When it is unnecessary to cleave the mass-changed ions 63, the processing unit 50 makes a negative determination on step S1007, and step S1011 is started.

In step S1009, the cooling gas/CID gas introduction unit 222 introduces a CID gas into the ion generation unit 20 and the ion generating unit 20 cleaves the mass-changed ions 63 by CID. After step S1009 is completed, step S1011 is started.

In step S1011, the time-of-flight mass separation unit 31 and the detection unit 32 detect ions resulting from dissociation of the mass-changed ions 63 or ions obtained by dissociation of the mass-changed ions 63 by mass analysis. After step S1011 is completed, step S1013 is started. In step S1013, the analysis unit 51 obtains a mass spectrum based on the measurement data of the mass analysis. After step S1013 is completed, step S1015 is started.

In step S1015, the analytical device 1 performs steps S1001 to S1011 described above separately without introducing oxygen gas into the ion generation unit 20 to obtain a mass spectrum. After step S1015 is completed, step S1017 is started. In step S1017, the analysis unit 51 identifies corresponding peaks in the mass spectrum obtained in step S1013 and the mass spectrum obtained in step S1015 based on the difference in mass between the radical ions 62 in step S1003 and the mass-changed ions 63 in step S1005 caused by the attachment of an oxygen atom contained in the oxygen gas. After step S1017 is completed, step S1019 is started.

In step S1019, the output unit 44 displays results of analysis on the mass spectra. After step S1019 is completed, the processing is terminated.

The embodiment described above provides the following effects.
(1) The analysis method according to the present embodiment comprises reacting ions S derived from a sample component with hydrogen radicals to generate radical ions 62; reacting an introduction gas with the radical ions 62 or ions generated by dissociation of the radical ions 62 to generate mass-changed ions 63; detecting the mass-changed ions 63 or ions generated by dissociation of the mass-changed ions 63 by mass analysis to obtain an introduction mass spectrum; and identifying peaks on the introduction mass spectrum based on differences in mass between the radical ions 62 and the mass-changed ions 63 on the basis of the composition of the introduction gas. This method enables the generated fragment ions to have different masses and enables detailed analysis of peaks on the obtained mass spectra by taking into consideration the resultant differences in mass.
(2) In the analysis method or analytical device 1 according to the present embodiment, the measurement unit 100 detects ions generated by dissociation of the radical ions 62 by mass analysis and the analysis unit 51 forms non-introduction mass spectrum and identifies corresponding peaks in the introduction mass spectrum and the non-introduction mass spectrum based on the difference in mass between the radical ions 62 and the mass-changed ions 63 on the basis of the composition of the oxygen gas. This construction makes it easier to identify the peak shifted along m/z direction by comparison between the introduction mass spectrum and the non-introduction mass spectrum.
(3) In the analysis method or analytical device 1 according to the present embodiment, the mass-changed ions 63 are generated by attachment of an oxygen atom contained in the introduction gas to each ion generated by dissociation of the radical ions 62 or the ions generated by dissociation of the radical ions 62. This construction enables efficient analysis of the mass spectra by using shift of the peak corresponding to a specified fragment ion according to the mass of the oxygen atom attached.
(4) In the analysis method or analytical device 1 according to the present embodiment, the sample component to be analyzed is at least one molecule selected from the group consisting of amino acid, peptide, and protein and the introduction gas comprises a molecule containing an oxygen atom. This construction enables efficient analysis of the mass spectra by utilizing shift of the peak corresponding to the fragment ion, which is obtained by cutting the main chain of the peptide or the like at a specified position, according to the mass of the molecule contained in the introduction gas.
(5) In the analysis method or analytical device 1 according to the present embodiment, the analysis unit 51 identifies a-type ions based on a difference in mass between the radical ion 62 and the mass-changed ion 63 caused by bonding with an oxygen atom. This construction enables efficient analysis of the mass spectra by using shift of the peak corresponding to the a-type fragment ion according to the mass of the oxygen atom contained in the introduction gas.
(6) In the analytical device 1 according to the present embodiment, hydrogen radical is used as the radical. The radical ions 62 are generated by attachment of the hydrogen radical to the ions S derived from the sample or by abstraction of hydrogen radical from the ions S derived from the sample. This enables the ions S derived from the sample to be converted into radicals by attachment or abstraction of the hydrogen radical to or from each ion S to increase reactivity, thus make it easier to cause various mass changes.
(7) The analytical device 1 according to the present embodiment comprises a radical introduction unit that introduces a hydrogen radical; an ion generation unit 20 that causes ions S derived from a sample component and the hydrogen radical to react with each other to generate radical ions 62 and causes an introduction gas and the radical ions 62 or ions generated by dissociation of the radical ions 62 to react with each other to generate mass-changed ions 63; a detection unit 32 that detects the mass-changed ions 63 or ions generated by dissociation of the mass-changed ions 63; and an oxygen gas introduction unit that is configured to be controllable separately from control of introduction of a cooling gas and/or CID gas and introduces the introduction gas to the ion generation unit 20. This configuration enables generated fragment ions to have different masses from each other and makes it possible to perform detailed analysis of the peaks on the mass spectra thus obtained by taking into consideration the resultant differences in mass.
(8) In the analysis method according to the present embodiment, the mass-changed ions 63 are generated by attachment of a portion of a molecule contained in the introduction gas to the radical ions 62 or the ions generated by dissociation of the radical ions 62. This enables efficient analysis of the mass spectra by using the shift of peaks of the fragment ions according to the mass of the portion of the introduction gas attached thereto.

The following variations are also within the scope of the present invention and may be combined with the above-mentioned embodiment. In the following variations, structures and sites that show functions similar to those in the above-mentioned embodiment are designated with the same reference symbols as those used in the above-mentioned embodiment and explanation thereof is omitted as appropriate.

### Variation 1

In the above-mentioned embodiment, explanation has been made of an example in which for a molecule constituted by a plurality of amino acids connected through peptide bonds, peaks are identified based on the regularity of cleavage position and a difference in mass caused by attachment of an oxygen atom. However, mass spectra may be analyzed by using attachment of an oxygen atom to a lipid.

FIG. 4 is a diagram illustrating a reaction in which an oxygen atom contained in the introduction gas is attached to a lipid. Here, a "lipid" refers to a molecule having a side chain containing a fatty acid or hydrocarbon moiety. A phospholipid 71, which is an ion S derived from a sample, includes a carbon atom designated by B0 on a side chain made of a glycerin as a backbone and fatty acids.

In the ion generation unit 20, the phospholipid 71 is converted into a radical by abstraction of hydrogen from the carbon B0 by a hydrogen radical to form a radical ion 72 (arrow B1). The radical-converted carbon B0 abstracts an oxygen atom contained in the introduction gas introduced into the ion generation unit 20 to form a single covalent bond with the abstracted oxygen atom. As a result, a mass-changed ion 73 is formed (arrow B2). The oxygen atom bonded to the carbon B0 has an unpaired electron and thus acts as a radical.

The time-of-flight mass separation unit 31 separates the mass-changed ion 73 or the ion generated by dissociation of the mass-changed ion 73 by mass analysis and the detection unit 32 detects the separated ions. The analysis unit 51 identifies peaks on the mass spectrum based on a difference in mass caused by attachment of the oxygen atom. The analysis unit 51 identifies the peaks that correspond to a molecule derived from a lipid by utilizing a shift of the peak on the mass spectra along m/z direction based on the mass of the oxygen atom between the mass spectra obtained with or without introduction of the oxygen gas.

In the analysis method according to the present variation, the sample component includes a side chain containing a fatty acid or hydrocarbon moiety and the introduction gas comprises a molecule that contains an oxygen atom. This construction enables efficient analysis of the mass spectra by using shift of the peak of a molecule derived from a lipid on the mass spectrum along m/z direction caused by the reaction between the oxygen atom and the radical ion 72.

In the analysis method according to the present variation, the radical ion 72 is identified to be derived from a lipid based on the difference in mass between the radical ion 72 and the mass-changed ion 73 caused by bonding with an oxygen atom. This configuration enables efficient analysis of the mass spectra by using shift of the peak of a molecule derived from a lipid on mass spectrum due to attachment of an oxygen atom to the radical ion 72.

### Variation 2

In the above-mentioned embodiment, the configuration is adopted in which radicals such as hydrogen radicals are introduced to the ion generation unit 20 to generate the radical ions 62 and 72. However, the ions S derived from a sample may also be converted into radicals by irradiation with a stream of electrons.

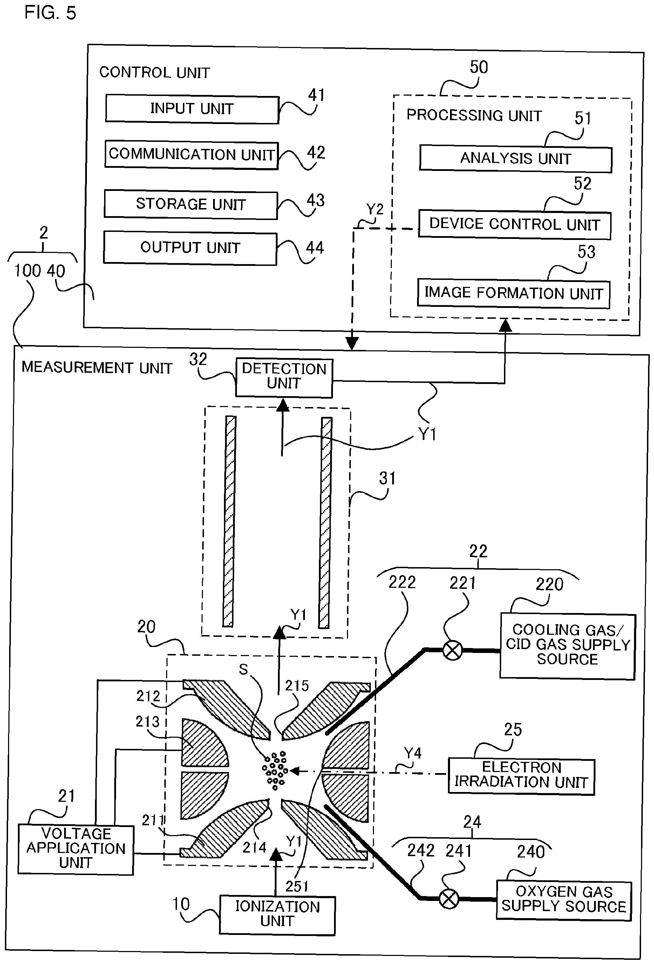

FIG. 5 is a diagram for illustrating a schematic configuration of the analytical device 2 according to the present variation. The analytical device 2 has a construction similar to that of the analytical device 1 except that the former includes an electron irradiation unit (electron irradiator) 25 instead of the radical supply unit 23 and also includes an electron introduction unit 251 instead of the radical introduction unit 216. A stream of electrons from the electron irradiation unit 25 is indicated by an arrow Y4 in dot-and-dash line.

The electron irradiation unit 25 includes an electron irradiation device that irradiates electrons by applying voltage to a filament to heat it and generate thermoelectrons and accelerating the generated thermoelectrons. The electron irradiation unit 25 irradiates ions S derived from the sample component with electrons having energy of preferably 50 eV or more, more preferably 500 eV or more. The electrons emitted from the electron irradiation unit 25 pass through the electron introduction unit 251 formed as an opening of the ring electrode 213 or the like and are irradiated to the ions S derived from the sample.

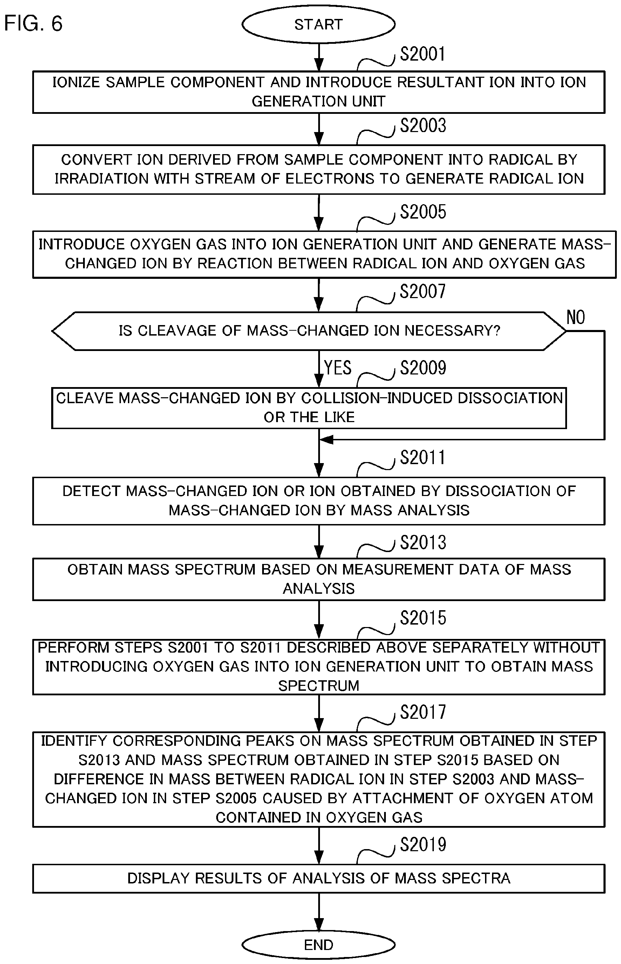

FIG. 6 is a flowchart illustrating the flow of the processing of the analysis method according to the present variation. Steps S2001 and S2005 to S2019 in the flowchart of the present variation are the same as steps S1001 and S1005 to S1019 in the flowchart of the above-mentioned embodiment (FIG. 3), respectively, and thus the explanation thereof are omitted.

In step S2003, the ion generation unit 20 converts ions derived from the sample component into radicals by irradiating the ions S derived from the sample component with a stream of electrons to generate radical ions 62 and 72. After step S2003 is completed, step S2005 is started.

The analytical device 1 according to the present embodiment includes an electron irradiation unit 25 that irradiates electrons and an ion generation unit 20 that irradiates ions derived from a sample component with electrons to generate radical ions 62 and 72, causes the radical ions 62 and 72 or ions generated by dissociation of the radical ions 62 and 72 to react with an introduction gas to generate mass-changed ions 63 and 73. This configuration makes a device that generates radicals unnecessary and makes it easier to generate ions having increased valences. This feature makes it possible to perform analysis taking advantage thereof and increase the efficiency of dissociation.

According to the present invention, fragment ions having mutually different masses can be generated by using a reaction with a radical and detailed analysis can be performed on peaks on the mass spectrum obtained by utilizing the differences in mass of the fragment ions.

The present invention is not limited to the above embodiment. Other embodiments contemplated in the technical idea of the present invention are also included within the scope of the present invention.

### EXAMPLES

### EXAMPLE 1

A sample containing ten (10) pmol of Substance P (1347Da), which is a peptide composed of 11 amino acids, was ionized by the MALDI method (matrix: CHCA) and measurement was performed in a positive ion mode. Mass spectroscopy was performed by using a mass spectrometer that was modified to include an ion trap and a time-of-flight mass separation unit to enable introduction of hydrogen radicals and an oxygen gas into the ion trap (the same is true for the examples described below).

Under a first condition, ions that were ionized by the MALDI method were introduced into the ion trap. Subsequently, an ion having a molecular weight of 1348 Da was separated in the ion trap as a monovalent precursor ion [M+H]⁺ (hereafter, "M" represents a sample component to be measured). Hydrogen radicals were irradiated to the separated precursor ions for 500 ms and the resultant fragment ions are mass-separated by the time-of-flight mass separation unit and then detected.

Under a second condition, the ions that were ionized by the MALDI method were introduced into the ion trap in the same manner as that under the first condition and subsequently ions having a molecular weight of 1348 Da were separated in the ion trap as monovalent precursor ions [M+H]⁺. The separated precursor ions were irradiated with hydrogen radicals for 500 ms, during which an oxygen gas equivalent to 1E⁻¹ [Pa] was introduced into the ion trap. An ion having a molecular weight of 1363 Da corresponding to the molecular weight of an ion derived from a sample from which hydrogen was abstracted and to which an oxygen atom was attached ([M+H-H+O]⁺) was separated in the ion trap, and fragment ions are generated from this ion by CID, mass-separated by a time-of-flight mass separation unit and then detected.

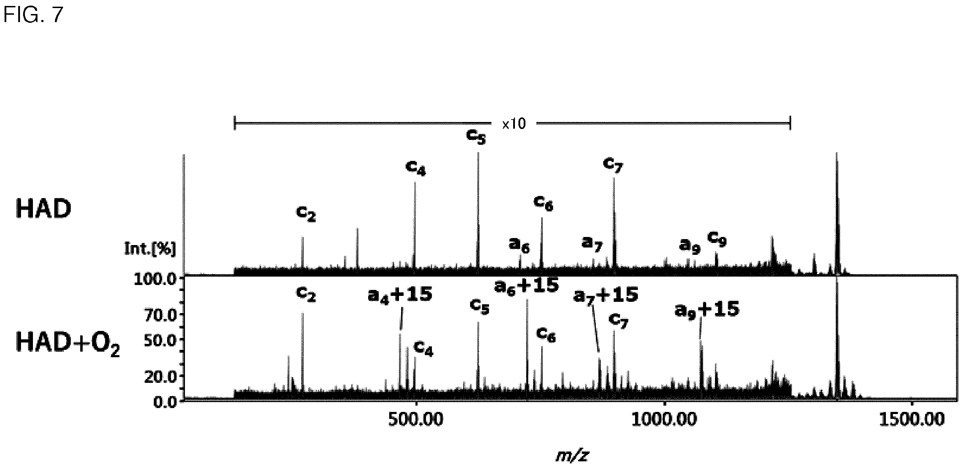

FIG. 7 is a chart of mass spectra showing results of measurement in Example 1. The result of the measurement under the first condition is a mass spectrum labeled HAD (Hydrogen Attachment/Abstraction Dissociation) in the upper column. The result of the measurement carried out under the second condition is a mass spectrum labeled "HAD+O₂" in the lower column. The mass spectrum obtained under the second condition under which an oxygen gas was introduced, an increase in molecular weight of 15 was observed for a-type ions. This increase corresponds to abstraction of a hydrogen atom and attachment of an oxygen atom.

### EXAMPLE 2

Under the first condition, a sample containing 10 pmol of phospholipid (PC(18:1(9Z)), 521 Da) was ionized and introduced into an ion trap. Subsequently, a monovalent phospholipid ion [M+H]⁺ was separated in the ion trap. The separated phospholipid ion was irradiated with hydrogen radicals for 500 ms and then mass-separated by a time-of-flight mass separation unit and then detected.

Under the second condition, a sample containing 10 pmol of a phospholipid (PC(18:1(9Z)), 521 Da) was ionized and introduced into an ion trap in the same manner as the first condition. Subsequently, a monovalent phospholipid ion [M+H]⁺ was separated in the ion trap. The separated phospholipid ion was irradiated with hydrogen radicals for 500 ms, during which an oxygen gas equivalent to 1E⁻¹ [Pa] was introduced into the ion trap. The generated ions were mass-separated by a time-of-flight mass separation unit and then detected.

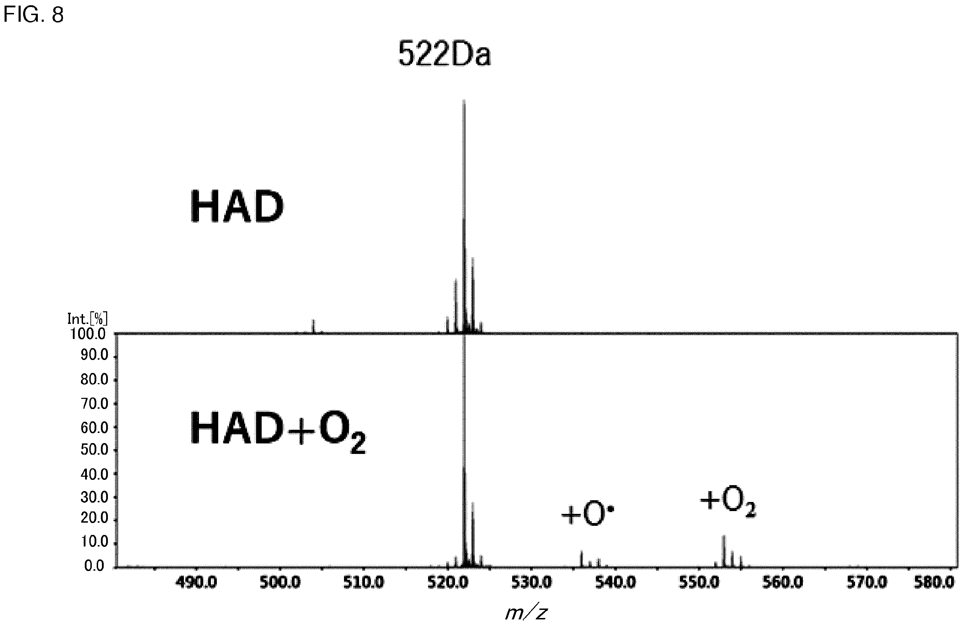

FIG. 8 is a chart of mass spectra showing results of measurement in Example 2. The result of measurement carried out under the first condition is shown by the upper column mass spectrum labeled "HAD". The result of measurement carried out under the second condition is shown by the lower column mass spectrum labeled "HAD+O₂". In the mass spectrum obtained under the first condition, ions (520 to 521 Da) from which the hydrogen atom was abstracted are observed. In the mass spectrum obtained under the second condition under which an oxygen gas was introduced, peaks corresponding to phospholipid ions to which one or more oxygen atoms were attached are observed.

### EXAMPLE 3

Under the first condition, substance P was ionized and introduced into the ion trap. Subsequently, monovalent ions [M+H]⁺ were separated in the ion trap. Electrons having 500 eV were irradiated to the separated ions for 500 ms and the resultant ions were mass-separated by a time-of-flight mass separation unit and then detected.

Under the second condition, like under the first condition, substance P was ionized and introduced into the ion trap. Subsequently, the resultant monovalent ion [M+H]⁺ was separated in the ion trap. Electrons having energy of 500 eV were irradiated to the separated ions for 500 ms, during which an oxygen gas equivalent to 1E⁻¹ [Pa] was introduced in the ion trap. The generated ions were mass-separated by a time-of-flight mass separation unit and then detected.

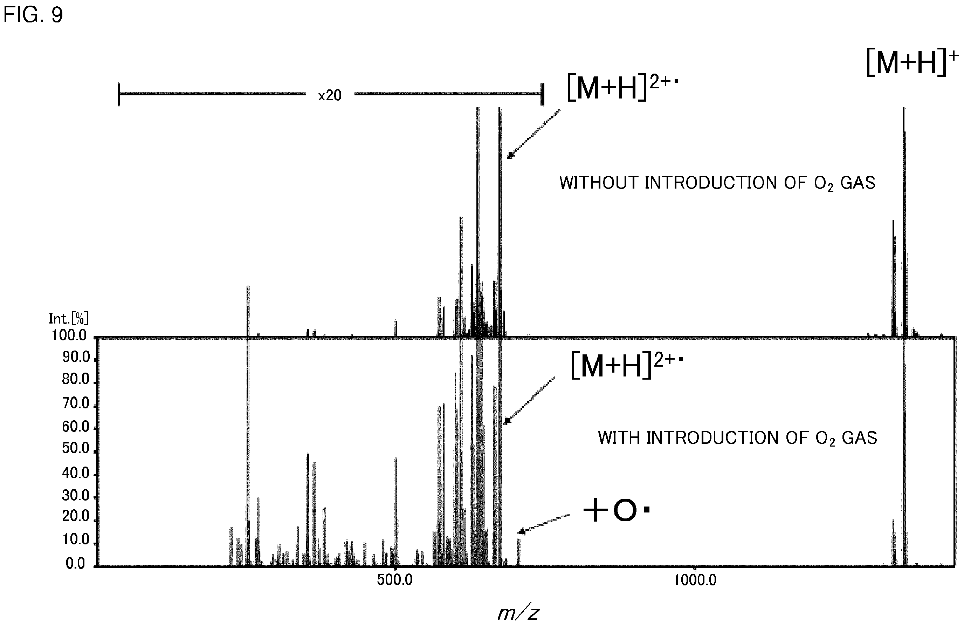

FIG. 9 is a chart of mass spectra showing results of measurement in Example 3. The result of measurement carried out under the first condition is shown by the upper column mass spectrum labeled "WITHOUT INTRODUCTION OF O₂ GAS". The result of measurement carried out under the second condition is shown by the lower column mass spectrum labeled "WITH INTRODUCTION OF O₂ GAS". In the mass spectra obtained under the respective conditions, radical ions [M+H]^{2+·} that have increased valences are observed. In the mass spectrum obtained under the second condition under which an oxygen gas was introduced, a peak corresponding to an ion derived from a radical ion to which an oxygen atom was attached is observed.

## Claims

1. An analysis method comprising:
converting a first ion derived from a sample component into a radical to generate a second ion;
reacting the second ion or an ion generated by dissociation of the second ion with an introduction gas to generate a third ion;
detecting the third ion or an ion generated by dissociation of the third ion by mass analysis to obtain a first mass spectrum: and
identifying a peak on the first mass spectrum based on a difference in mass between the second ion or the ion generated by dissociation of the second ion and the third ion or the ion generated by dissociation of the third ion due to the composition of the introduction gas.

2. The analysis method according to claim 1, further comprising:
detecting the ion generated by dissociation of the second ion by mass analysis and forming a second mass spectrum; and
identifying corresponding peaks in the first mass spectrum and the second mass spectrum based on a difference in mass between the second ion or the ion generated by dissociation of the second ion and the third ion or the ion generated by dissociation of the third ion due to the composition of the introduction gas.

3. The analysis method according to claim 1 or 2, wherein:
the third ion or the ion generated by dissociation of the third ion is generated by attachment of a portion of a molecule contained in the introduction gas to the second ion or the ion generated by the second ion.

4. The analysis method according to any one of claims 1 to 3, wherein:
the sample component is at least one molecule selected from the group consisting of amino acid, peptide, and protein; and
the introduction gas contains a molecule that comprises an oxygen atom;
optionally wherein:
an a-type ion is identified based on a difference in mass between the second ion or the ion generated by dissociation of the second ion and the third ion or the ion generated by dissociation of the third ion due to attachment of the oxygen atom.

5. The analysis method according to any one of claims 1 to 3, wherein:
the sample component comprises a side chain that contains a fatty acid or hydrocarbon moiety; and
the introduction gas contains a molecule that comprises an oxygen atom;
optionally wherein:
the sample component is identified to be derived from a lipid based on a difference in mass between the second ion or the ion generated by dissociation of the second ion and the third ion or the ion generated by dissociation of the third ion due to attachment of the oxygen atom.

6. The analysis method according to any one of claims 1 to 5, wherein:
the first ion is converted to a radical by reacting the first ion with a gas containing a radical;
optionally wherein:
the radical is a hydrogen radical, and
the second ion is generated by attachment of the hydrogen radical to the first ion or by abstraction of the hydrogen radical from the first ion.

7. The analysis method according to any one of claims 1 to 4, wherein:
the first ion is converted into a radical by irradiating the first ion with electrons.

8. The analysis method according to any one of claims 1 to 7, wherein
the introduction gas contains an oxygen molecule.

9. The analysis method according to any one of claims 1 to 8, further comprising:
dissociating the third ion by any one of methods consisting of gas impact dissociation, laser irradiation dissociation, electronic ionization dissociation, electron transfer dissociation, and dissociation by attachment or abstraction of hydrogen to generate fragment ions.

10. An analytical device comprising:
an ion generation unit that converts a first ion derived from a sample component into a radical to generate a second ion, reacts the second ion or an ion generated by dissociation of the second ion with an introduction gas to generate a third ion or an ion generated by dissociation of the third ion;
a detection unit that detects the third ion or the ion generated by the third ion by mass analysis;
a first gas introduction unit that introduces a cooling gas and/or CID gas to the ion generation unit; and
a second gas introduction unit that is configured to be controllable independently from control of the cooling gas and/or CID gas and introduces the introduction gas to the ion generation unit.

11. The analytical device according to claim 10, further comprising:
an analysis unit that identifies a peak in a mass spectrum detected by the mass analysis based on a difference in mass between the second ion or the ion generated by dissociation of the second ion and the third ion or the ion generated by dissociation of the third ion due to the composition of the introduction gas.

12. The analytical device according to claim 10 or 11, further comprising:
a radical introduction unit that introduces a radical, wherein
the ion generation unit converts the first ion into a radical by reaction of the first ion with a gas containing a radical.

13. The analytical device according to claim 12, wherein
the radical is a hydrogen radical, and
the second ion is generated by attachment of the hydrogen radical to the first ion or by abstraction of the hydrogen radical from the first ion.

14. The analytical device according to claim 10 or 11, further comprising:
an electron irradiation unit that irradiates electrons, wherein
the ion generation unit converts the first ion into a radical by irradiating the first ion with electrons.

15. The analytical device according to any one of claims 10 to 14, wherein
the introduction gas contains a molecule that comprises an oxygen atom.
